# EUROPÄISCHE PATENTSCHRIFT

(11) **EP 1 184 001 B1**
(45) Veröffentlichungstag und Bekanntmachung des Hinweises auf die Patenterteilung: **19.11.2003**
(21) Anmeldenummer: 00118483.7
(22) Anmeldetag: 25.08.2000
(51) Int. Cl.: A61B 17/66, A61C 8/00

(54) **Vorrichtung zur Distraktion von Knochensegmenten**
Device for distracting bone segments
Dispositif pour la distraction de segments osseux

(43) Veröffentlichungstag der Anmeldung: 06.03.2002
(73) Patentinhaber: FRIADENT GmbH, 68229 Mannheim (DE)
(72) Erfinder: Neugebauer, Jörg, Dr., 69121 Heidelberg (DE); Vizethum, Freimut, Dr. Dipl.-Ing., 68723 Schwetzingen (DE)
(74) Vertreter: Schmitt, Meinrad, Dipl.-Ing.

(56) Entgegenhaltungen:
- WO-A-00/41637
- US-A- 6 001 101
- US-A- 6 050 819

## Beschreibung

Die Erfindung bezieht sich auf eine Vorrichtung zur Distraktion von Knochensegmenten.

Aus der EP 0 770 359 A 1 ist eine derartige Vorrichtung bekannt, welche relativ zueinander verstellbare Halterungen zur Distraktion von Knochensegmenten im Kiefer-, Gesichts- und Schädelbereich enthält. Die Halterung enthält zwei komplementäre Hälften, welche mittels einer Einstellvorrichtung längs einer geometrischen und zumindest im wesentlichen linearen Längsachse unter Vergrößerung ihres gegenseitigen Abstandes bewegbar sind. Diese Hälften sind durch eine verdrehsichere Führung aneinander längsbeweglich geführt und enthalten jeweils zumindest eine flache, am Knochen fixierbare Fixiervorrichtung zur Aufnahme von Schubkräften und Drehmomenten. Die genannte Einstellvorrichtung umfaßt mit einer Hälfte eine Hülse, deren Endbereich zumindest teilweise offen ist, wobei in dem Endbereich ein Führungsabschnitt drehbar gelagert ist. Ferner ist außerhalb des genannten Endbereiches eine an dem Führungsabschnitt angeordnete Werkzeugaufnahme vorgesehen. Der Fertigungsaufwand dieser Vorrichtung ist nicht unerheblich, und im Hinblick auf das Bauvolumen kann die operative Handhabung zu Problemen führen.

Eine weitere Distraktions vorrichtung ist aus der US-A-6 050 819 bekannt.

Hiervon ausgehend liegt der Erfindung die Aufgabe zugrunde, die Vorrichtung dahingehend weiterzubilden, daß bei einfacher Konstruktion und einfacher Handhabung ein optimiertes Knochenwachstum und eine schnelle Knochenregeneration ermöglicht wird. Die Vorrichtung soll mit einem geringen Fertigungsaufwand realisierbar sein und eine möglichst geringe Belastung des Patienten erfordern.

Die Lösung dieser Aufgabe erfolgt gemäß den im Patentanspruch 1 angegebenen Merkmalen.

Die erfindungsgemäße Distraktionsvorrichtung enthält eine Distraktionsschraube mit einem vergleichsweise groben Gewinde und einer in zweckmäßiger Weise stumpfen bzw. abgerundeten Spitze sowie eine Führungsschraube mit einem selbstbohrenden oder selbstschneidenden Gewinde. Die Gewindeflanken der Distraktionsschraube weisen eine Höhe in der Größenordnung von zwei Dritteln des Kerndurchmessers der Distraktionsschraube auf, wobei die Gewindespitze an der Spitze der Distraktionsschraube stumpf ausgebildet ist. Die Spitze der Distraktionsschraube weist in bevorzugter Weise einen Durchmesser gleich oder kleiner als der Kerndurchmesser auf. Die Führungsschraube ist mit einem selbstbohrenden oder selbstschneidenden Gewinde ausgebildet und enthält in vorteilhafter Weise einen sich zum Ende konisch reduzierten Kopf. Des weiteren enthält die Führungsschraube einen achsparallelen Bereich oder Knochenanteil und / oder einen konischen Führungsanteil, dessen Konuswinkel bevorzugt größer als ein Winkelgrad ist. Die erfindungsgemäße Vorrichtung dient insbesondere zur Callus-Distraktion zwecks Vergrößerung des Alveolarfortsatzes. Hierzu wird ein vorgegebener Bereich unterhalb des Knochenkamms des Kieferknochens von diesem zumindest teilweise getrennt, wobei in der nachfolgend zu erläuternden Weise mittels der Distraktionsschraube in Kombination mit der Führungsschraube sukzessive eine definierte Vergrößerung des Abstandes des abgetrennten Knochensegments bezüglich des Kieferknochens derart durchgeführt wird, daß zur Alveolarfortsatz-Distraktion sich im Trennbereich neues Knochengewebe bildet, welches das abgetrennte Knochensegment fest mit dem Kieferknochen verbindet. Mittels der Führungsschraube erfolgt hierbei eine definierte Führung, wobei aufgrund des in bevorzugter Weise konischen Führungsanteils oder Bereiches unerwünschte und für das Wachstum nachteilige Spannungen vermieden werden. Es erfolgt in vorteilhafter Weise eine Aufteilung der Funktionen derart, daß einerseits durch Drehen der auf dem Knochenkamm, welcher nachfolgend auch als erstes Knochensegment bezeichnet wird, abgestützten Distraktionsschraube der Abstand des abgetrennten bzw. zweiten Knochensegments bzw. der Trennbereich vergrößert wird und daß ferner andererseits die erforderliche Führung des zweiten Knochensegments bezüglich des ersten Knochensegments gewährleistet ist. Die Verwendung der erfindungsgemäßen Distraktionsvorrichtung ist jedoch keinesfalls auf die Vergrößerung des Alveolarfortsatzes beschränkt, sondern kann in entsprechender Weise auch zur Distraktion von anderen Knochen bzw. Knochenteilen zum Einsatz gelangen.

Besondere Ausgestaltungen der Erfindung sind in den Unteransprüchen und der weiteren Beschreibung angegeben.

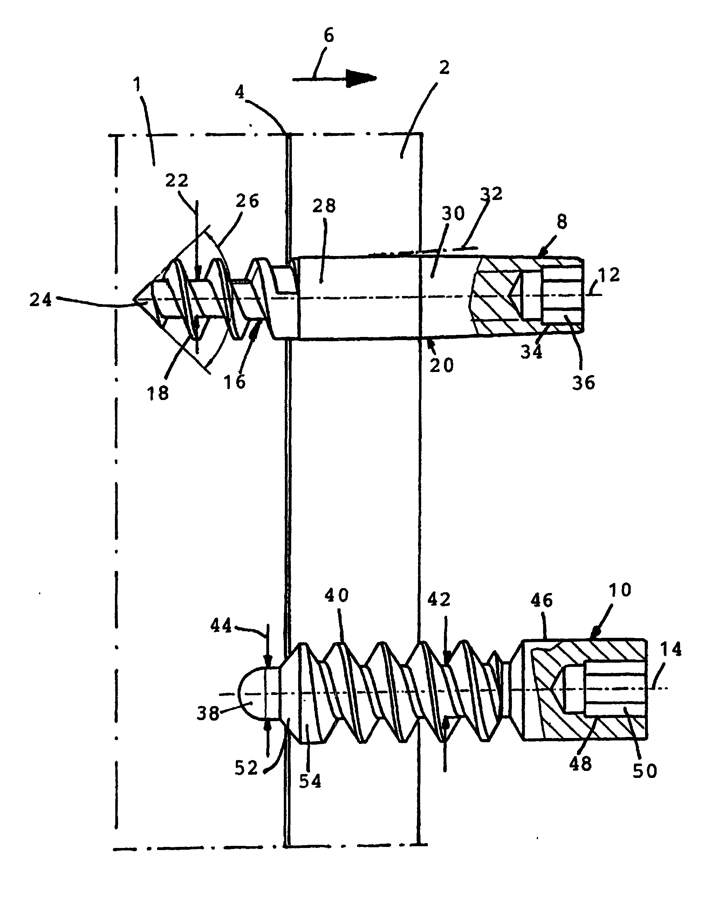

Die Erfindung wird nachfolgend an Hand eines besonderen in der Zeichnung dargestellten Ausführungsbeispiels näher erläutert, ohne daß insoweit eine Beschränkung erfolgt.

In der Zeichnung sind schematisch zwei Knochensegmente 1, 2 dargestellt, welche entlang eines Trennbereiches 4 voneinander getrennt sind. Vor und / oder hinter der Zeichenebene sowie außerhalb des dargestellten Teilbereiches der Knochensegmente 1, 2 ist wenigstens ein weiterer Trennbereich derart vorhanden, daß bezüglich des ersten Knochensegments 1 das zweite Knochensegment 2 in Richtung des Pfeiles 6 grundsätzlich bewegbar ist. So kann das Knochensegment 1 der Kieferknochen sein, von welchem das Knochensegment 2 in der angesprochenen Weise entlang der erwähnten Trennbereiche abgetrennt ist.

Die erfindungsgemäße Vorrichtung enthält in Kombination wenigstens zwei unterschiedlich ausgebildete Schrauben, und zwar eine Führungsschraube 8 und eine Distraktionsschraube 10, deren Längsachsen 12 bzw. 14 im wesentlichen parallel zueinander angeordnet sind. Des weiteren stehen die Achsen 12, 14 bevorzugt zumindest näherungsweise orthogonal zum Trennbereich 4.

Die Führungsschraube 8 enthält einen apikalen Teil 16 mit einem selbstbohrenden oder selbstschneidenden Gewinde 18 zum Einschrauben In das erste Knochensegment 1 sowie einen Führungsteil 20, welcher dem zweiten Knochensegment 2 zugeordnet ist. Die Führungsschraube 8 wird durch das zweite Knochensegment 2 durchgeführt und mittels des Gewindes 18 im ersten Knochensegment 1 verankert. Der Außendurchmesser des Gewindes 18 ist maximal gleich groß wie der Außendurchmesser des Führungsteiles 20. Bevorzugt wird in das zweite Knochensegment 2 zum Durchführen der Führungsschraube 8 eine Bohrung eingebracht, deren Innendurchmesser im wesentlichen dem Außendurchmesser des Führungsteiles 20 entspricht. Alternativ kann zumindest in das zweite Knochensegment 2 eine Pilotbohrung eingebracht werden, mit einem Durchmesser zumindest näherungsweise gleich groß wie der Kerndurchmesser 22 des apikalen Teils 16 bzw. des Gewindes 18. Am apikalen Ende besitzt die Führungsschraube 8 eine Spitze 24, weiche insbesondere konisch ausgebildet ist, mit einem Öffnungswinkel 26 im Bereich zwischen 70° bis 110°, insbesondere näherungsweise von 90°. Der Führungsteil 20 weist anschließend an das Gewinde einen im wesentlichen zylindrischen Bereich 28 auf und ferner einen konischen Führungsbereich 30, dessen Durchmesser, ausgehend vom zylindrischen Bereich 28, sich verjüngt. Der Konuswinkel des konischen Führungsbereiches 13 ist in bevorzugter Weise gleich oder größer 1° vorgegeben. Alternativ kann im Rahmen der Erfindung der Führungsteil 20 auch durchgehend zylindrisch ausgebildet sein, wobei in das Knochensegment 2 entsprechend der strichpunktierten Linie 32 eine sich konisch erweiternde Bohrung eingebracht wird, welche sich in der Richtung weg vom apikalen Teil 16 erweitert, und zwar bevorzugt mit einem Konuswinkel gleich oder größer 1°. Festzuhalten bleibt, daß die letztgenannte Bohrung einen dem apikalen Teil 16 benachbarten zylindrischen Teil aufweist, an welchem mit größerem Abstand zum apikalen Teil 16 die konische Erweiterung anschließt. Der Führungsteil 20 enthält im freien Ende eine Ausnehmung 34 mit insbesondere als Hexagon ausgebildeten Eingriffsflächen 36 für ein korrespondierend ausgebildetes Eindrehwerkzeug.

Wie in der Zeichnung dargestellt, ist beabstandet zur Führungsschraube 8 die Distraktionsschraube 10 angeordnet, welche eine stumpfe, bevorzugt abgerundete Spitze 38 besitzt. Die Spitze 38 dient zur Auflagerung der Distraktionsschraube 10 bezüglich des ersten Knochensegments 1. Die Distraktionsschraube 10 enthält anschließend an die Spitze 8 ein Gewinde 40, welches dem zweiten Knochensegment 2 zugeordnet ist und dieses durchdringt. Es handelt sich hierbei um ein vergleichsweise grobes Gewinde, wobei die Flankenhöhe zumindest näherungsweise zwei Drittel des Kerndurchmessers 42 des Gewindes 40 beträgt. Der Durchmesser 44 der Spitze 38 ist in bevorzugter Weise gleich oder kleiner als der Kerndurchmesser 42. Ferner enthält die Distraktionsschraube 10 einen insbesondere zylindrischen Kopf 46 mit einer Ausnehmung 48, welche wiederum bevorzugt als Hexagon ausgebildete Eingriffsflächen 50 für ein Eindrehwerkzeug aufweist.

In bevorzugter Weise ist zwischen der Spitze 38 und dem Gewinde 40 ein insbesondere konisch zum Gewinde 40 erweiterter Bereich 52 vorgesehen, welcher eine sichere Abstützung der Distraktionsschraube 10 beim Drehen sicherstellt. Anschließend an den erweiterten Bereich 52 zum Gewinde 40 hin ist ferner ein ringförmiger Bereich 54 vorgesehen, welcher in Umfangsrichtung in besonders zweckmäßiger Weise geschlossen ausgebildet ist. Das Gewinde 40 bzw. der Kernbereich desselben mündet also nicht unmittelbar an der Spitze 38, sondern ist von dieser durch den erweiterten Bereich 52 und / oder den ringförmigen Bereich 54 getrennt. Aufgrund der über den Umfang geschlossenen Bereiche 52 und/oder 54 wird beim Drehen der Distraktionsschraube 10 deren Eindrehen in das erste Knochensegment 1 unterbunden. Somit behält die Distraktionsschraube 10 während der Distraktion ihre axiale Position bezüglich des ersten Knochensegmentes 1 bei, während hingegen das zweite Knochensegment 2 durch Drehen der Distraktionsschraube 10 in Richtung des Pfeiles 6 vom ersten Knochensegment wegbewegt wird. Das Gewinde 40 endet somit stumpf an dem Bereich 54 und /oder an dem erweiterten Bereich 52, ohne diese jedoch axial zu durchdringen.

In der dargestellten Anfangsposition nach dem Verankern der Führungsschraube 8 im ersten Knochensegment 1 und Einschrauben der Distraktionsschraube 10 wird zu Beginn der Distraktion mittels der Führungsschraube 8 eine exakte parallele Führung aufgrund des zylindrischen Bereichs 28 des Führungsteils 20 der Führungsschraube 8 gewährleistet. Mit zunehmender Vergrößerung des Abstandes des zweiten Knochensegments 2 bezüglich des ersten Knochensegments 1 in Richtung des Pfeiles 6 wird aufgrund des konischen Führungsbereiches 30 und / oder der konisch erweiterten Bohrung die Führung reduziert, so daß ein hinreichender Ausgleich ermöglicht wird und nachteilige Auswirkungen in dem entsprechend der Distraktion vergrößerten Trennbereich der beiden Knochensegmente 1, 2 wesentlich reduziert werden und das Knochenwachstum optimiert wird.

### Bezugszeichen

- 1, 2: Knochensegment
- 4: Trennbereich
- 6: Pfeil
- 8: Führungsschraube
- 10: Distraktionsschraube
- 12: Längsachse von 8
- 14: Längsachse von 10
- 16: apikaler Teil von 8
- 18: Gewinde
- 20: Führungsteil
- 22: Kerndurchmesser von 18
- 24: Spitze von 8
- 26: Öffnungswinkel
- 28: zylindrischer Bereich von 20
- 30: konischer Führungsbereich von 20
- 32: strichpunktierte Linie
- 34: Ausnehmung in 20
- 36: Eingriffsfläche
- 38: Spitze von 10
- 40: Gewinde von 10
- 42: Kerndurchmesser von 40
- 44: Durchmesser von 38
- 46: Kopf von 10
- 48: Ausnehmung in 46
- 50: Eingriffsfläche
- 52: erweiterter Bereich
- 54: ringförmiger Bereich

## Patentansprüche

1. Vorrichtung zur Distraktion von Knochensegmenten, wobei eine Führungsschraube (8) und eine Distraktionsschraube (10) vorgesehen sind, daß die Führungsschraube (8) ein im ersten Knochensegment (1) verankerbares Gewinde (18) und einen das zweite Knochensegment (2) durchdringenden Führungsteil (20) aufweist, und daß die Distraktionsschraube (10) eine in oder auf dem ersten Knochensegment (1) abstützbare Spitze (38) und ein dem zweiten Knochensegment (2) zugeordnetes Gewinde (40) aufweist, mittels welchem durch Drehen der Distraktionsschraube (10) der Trennbereich (4) zwischen den beiden Knochensegmenten (1, 2) vergrößerbar ist.

2. Vorrichtung nach Anspruch 1, **dadurch gekennzeichnet, daß** das Gewinde (18) der Führungsschraube (8) selbstbohrend oder selbstschneidend ausgebildet ist.

3. Vorrichtung nach Anspruch 1 oder 2, **dadurch gekennzeichnet, daß** der Führungsteil (20) der Führungsschraube (8) einen an das Gewinde (18) anschließenden im wesentlichen zylinderischen Bereich (28) und an diesen anschließend einen sich verjüngenden konischen Führungsbereich (30) enthält, wobei der Konuswinkel bevorzugt gleich oder größer 1 ° ist.

4. Vorrichtung nach einem der Ansprüche 1 bis 3, **dadurch gekennzeichnet, daß** das Gewinde (40) der Distraktionsschraube (10) grob ausgebildet ist und/oder dessen Gewindeflanken eine Höhe in der Größenordnung von zwei Dritteln des Kerndurchmessers aufweisen.

5. Vorrichtung nach einem der Ansprüche 1 bis 4, **dadurch gekennzeichnet, daß** die Spitze (38) der Distraktionsschraube (10) einen Durchmesser gleich oder kleiner als der Kerndurchmesser (42) aufweist.

6. Vorrichtung nach einem der Ansprüche 1 bis 5, **dadurch gekennzeichnet, daß** das Gewinde (40) der Distraktionsschraube (10) zur Spitze (38) hin stumpf ausläuft.

7. Vorrichtung nach einem der Ansprüche 1 bis 6, **dadurch gekennzeichnet, daß** zwischen der Spitze (38) und dem Gewinde (40) der Distraktionsschraube (10) ein erweiterter, bevorzugt konisch erweiterter Bereich (52) vorgesehen ist, welcher über den Umfang geschlossen ausgebildet ist, und / oder daß zwischen der Spitze (38) und der Gewindespitze ein über den Umfang zumindest näherungsweise geschlossener ringförmiger Bereich (54) vorgesehen ist, dessen Außendurchmesser im wesentlichen dem Außendurchmesser des Gewindes (40) entspricht.

## Claims

1. Device for distracting bone segments, wherein a guide screw (8) and a distraction screw (10) are provided, in that the guide screw (8) has a thread (18) which can be secured in the first bone segment (1) and a guide part (20) penetrating the second bone segment (2), and in that the distraction screw (10) has a tip (38) which can be supported in or on the first bone segment (1) and a thread (40) associated with the second bone segment (2), by means of which the separating region (4) between the two bone segments (1, 2) can be increased by turning the distraction screw (10).

2. Device according to claim 1, **characterised in that** the thread (18) of the guide screw (8) is designed to be self-tapping or self-cutting.

3. Device according to claim 1 or 2, **characterised in that** the guide part (20) of the guide screw (8) contains a cylindrical region (28) substantially adjacent to the thread (18) and contains a tapering conical guide region (30) adjacent to this region, the cone angle preferably being equal to or greater than 1°.

4. Device according to any one of claims 1 to 3, **characterised in that** the thread (40) of the distraction screw (10) is coarsely designed and/or the flanks of the thread have a height approximately two thirds of the core diameter.

5. Device according to any one of claims 1 to 4, **characterised in that** the tip (38) of the distraction screw (10) has a diameter which is equal to or smaller than the core diameter (42).

6. Device according to any one of claims 1 to 5, **characterised in that** the thread (40) of the distraction screw (10) extends bluntly toward the tip (38).

7. Device according to any one of claims 1 to 6, **characterised in that** provided between the tip (38) and the thread (40) of the distraction screw (10) is a widened, preferably conically widened region (52) which is designed so as to be closed over the periphery and/or **in that** provided between the tip (38) and thread tip is an annular region (54), which is at least virtually closed over the periphery, of which the external diameter substantially corresponds to the external diameter of the thread (40).

## Revendications

1. Dispositif pour la distraction de segments osseux, une vis de guidage (8) et une vis de distraction (10) étant prévues, ***caractérisé en ce que*** la vis de guidage (8) présente un filetage (18) pouvant être ancré dans le premier segment osseux (1) et une partie de guidage (20) traversant le deuxième segment osseux (2), et ***en ce que*** la vis de distraction (10) présente une pointe (38) pouvant s'appuyer dans ou sur le premier segment osseux (1) et un filetage (40) associé au deuxième segment osseux (2), au moyen duquel la partie de séparation (4) entre les deux segments osseux (1, 2) peut être agrandie par rotation de la vis de distraction (10).

2. Dispositif selon la revendication 1, ***caractérisé en ce que*** le filetage (18) de la vis de guidage (8) est autotaraudeur ou autoperçant.

3. Dispositif selon la revendication 1 ou 2, ***caractérisé en ce que*** la partie de guidage (20) de la vis de guidage (8) comprend une partie sensiblement cylindrique (28) faisant suite au filetage (18) et une partie de guidage (30) conique effilée lui faisant suite, l'angle de cône étant de préférence supérieur ou égal à 1°.

4. Dispositif selon l'une quelconque des revendications 1 à 3, ***caractérisé en ce que*** le filetage (40) de la vis de distraction (10) est grossier et/ou ses flancs de filetage présentent une hauteur de l'ordre des deux tiers du diamètre de l'âme.

5. Dispositif selon l'une quelconque des revendications 1 à 4, ***caractérisé en ce que*** la pointe (38) de la vis de distraction (10) présente un diamètre supérieur ou égal au diamètre d'âme (42).

6. Dispositif selon l'une quelconque des revendications 1 à 5, ***caractérisé en ce que*** le filetage (40) de la vis de distraction (10) s'effile de manière émoussée en direction de la pointe (38).

7. Dispositif selon l'une quelconque des revendications 1 à 7, ***caractérisé en ce qu*'**entre la pointe (38) et le filetage (40) de la vis de distraction (10), il est prévu une autre partie (52) élargie, de préférence conique, qui est fermée sur la circonférence, et/ou ***en ce qu*'**entre la pointe (38) et la pointe du filetage, il est prévu une partie annulaire (54) au moins approximativement fermée sur la circonférence, dont le diamètre extérieur correspond pour l'essentiel au diamètre extérieur du filetage (40).
